# EUROPÄISCHE PATENTANMELDUNG

(11) **EP 1 293 493 A2**
(43) Veröffentlichungstag der Anmeldung: **19.03.2003**
(21) Anmeldenummer: 02019407.2
(22) Anmeldetag: 30.08.2002
(51) Int. Cl.: C07C 35/18, C07C 69/013, C07C 47/21, C07C 43/196

(54) **Verfahren zur Herstellung von Meso-Zeaxanthin**

(30) Priorität: 13.09.2001 DE 10145223
(71) Anmelder: BASF AKTIENGESELLSCHAFT, 67056 Ludwigshafen (DE)
(72) Erfinder: Ernst, Hansgeorg, Dr., 67346 Speyer (DE); Henrich, Klaus, 67454 Hassloch (DE); Ditrich, Klaus, Dr., 67161 Gönnheim (DE)

(57) **Zusammenfassung**

Die Erfindung betrifft ein Verfahren zur Herstellung von optisch reinen Acetylendiolen der Formeln R-I und S-I 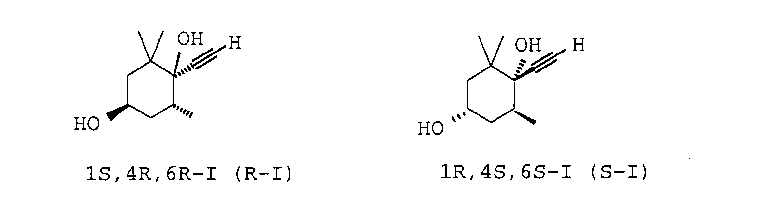 und deren weitere Umsetzung zu meso-Zexanthin.

## Beschreibung

Die vorliegende Erfindung betrifft ein neues Verfahren zur Herstellung von meso-Zeaxanthin. Meso-Zeaxanthin ist u.a für die Therapie und Prophylaxe von altersbedingter Maculadegeneration (AMD) von großer Bedeutung.

Die Altersblindheit als Folge der altersbedingten Maculadegeneration ist aus epidemiologischer Sicht ein bedeutendes Problem. Neuere Untersuchungen zeigen, dass bestimmte Carotinoide das Auge wirksam vor AMD und damit vor Erblindung schützen können. Die Carotinoide, die diese Schutzfunktion ausüben, sind Lutein und Zeaxanthin.

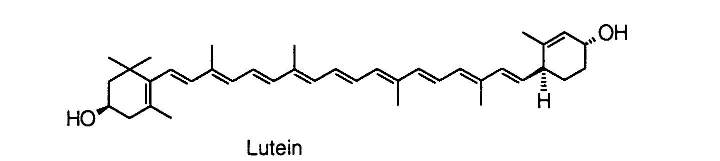

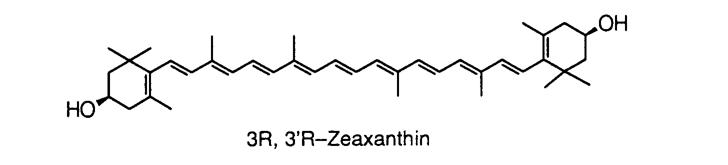

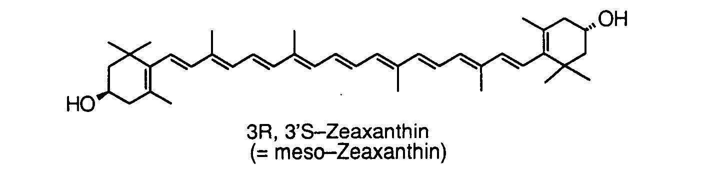

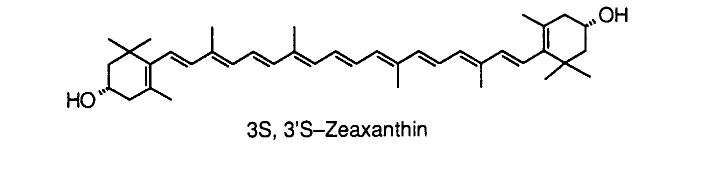

Lutein und Zeaxanthin können sowohl für die Prophylaxe als auch für die Behandlung fortgeschrittener AMD eingesetzt werden. Als besonders wirksam wurde die Verabreichung von meso-Zeaxanthin und Lutein beschrieben (US 6,218,436). Für diese therapeutische Aufgabe muss meso-Zeaxanthin zur Verfügung gestellt werden. Da eine Isolierung aus natürlichen Quellen ausscheidet, kommen nur Partialsynthesen (Isomerisierung von Lutein) oder totalsynthetische Verfahren in Betracht.

Es hat nicht an Versuchen gefehlt, Lutein durch basenkatalysierte Isomerisierung in meso-Zeaxanthin zu überführen (EP-A-0 834 536; WO 9602594; US 5,523,434). Die hier beschriebenen Verfahren zur Lutein-Isomerisierung führen stets zu Gemischen aus Lutein und meso-Zeaxanthin. Ein für therapeutische Zwecke gewünschtes einheitliches Produkt kann aus solchen Gemischen nur durch äußerst aufwendige Trennoperationen, verbunden mit hohen Ausbeuteverlusten gewonnen werden.

Eine vielstufige Totalsynthese von meso-Zeaxanthin, ausgehend von Safranal ist beschrieben in Pure Appl. Chem. 51, 535 f. (1979), Pure Appl. Chem. 51, 565 f. (1979), Helv. Chim. Acta 63, 6, 1377, (1980) und Helv. Chim. Acta 63, 6, 1465, (1980).

Die hier erzielten Ausbeuten an meso-Zeaxanthin sind für eine technische Durchführung der Synthese zu gering. Um ein einheitliches Endprodukt zu erhalten, ist es aufgrund der geringen Selektivitäten vieler Reaktionsschritte erforderlich, viele der anfallenden Zwischenprodukte aufwendig aufzureinigen.

Es war daher die Aufgabe der vorliegenden Erfindung, ein Verfahren zur Herstellung von meso-Zeaxanthin bereitzustellen, mit dem die oben genannten Nachteile des Standes der Technik vermieden werden.

Diese Aufgabe wurde gelöst durch ein Verfahren zur Herstellung von meso-Zexanthin, 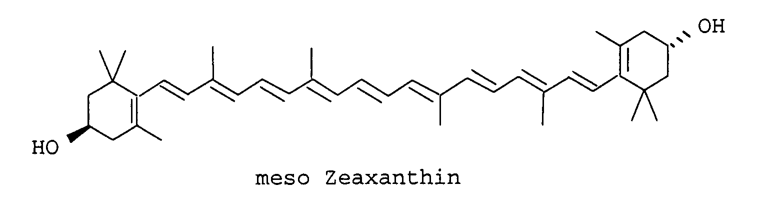 dadurch gekennzeichnet, dass man
a) ein racemisches Gemisch der Acetylendiole R-I und S-I 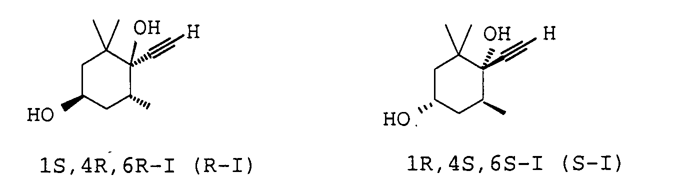 in seine Antipoden auftrennt,
b) die getrennten Antipoden R-I und S-I jeweils in die C₁₅-Phosphoniumsalze R-II bzw. S-II 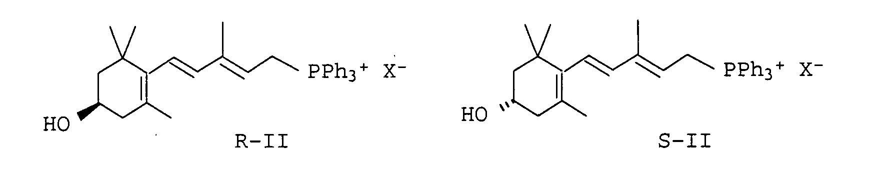 überführt, in denen Ph für Aryl und X für ein Anionenäquivalent einer anorganischen oder organischen Säure stehen,
c) die Phosphoniumsalze R-II oder S-II mit einem C₁₀-Dial-monoacetal der allgemeinen Formel III, 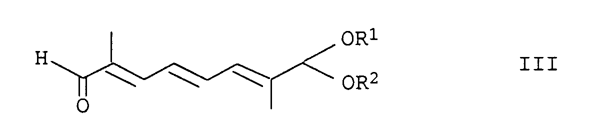 in der die Substituenten R¹ und R² unabhängig voneinander für C₁-C₈-Alkyl stehen oder gemeinsam mit den Sauerstoffatomen und dem Kohlenstoffatom, an denen sie gebunden sind einen 1,3-Dioxolan- oder 1,3-Dioxan-Ring der folgenden Strukturen bilden können, 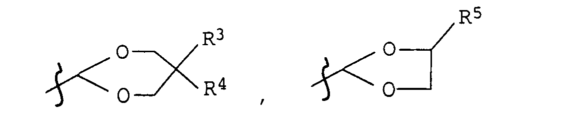 in denen R³ und R⁴ sowie R⁵ jeweils unabhängig voneinander Wasserstoff oder C₁-C₄-Alkyl bedeuten können, in einer Wittig-Reaktion zu den C₂₅-Acetalen R-IV oder S-IV umsetzt, 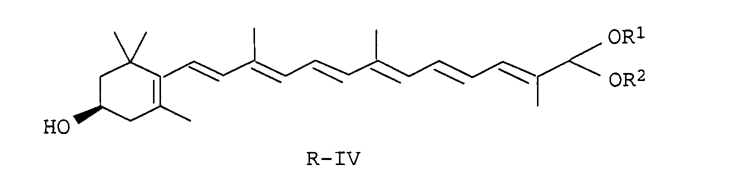 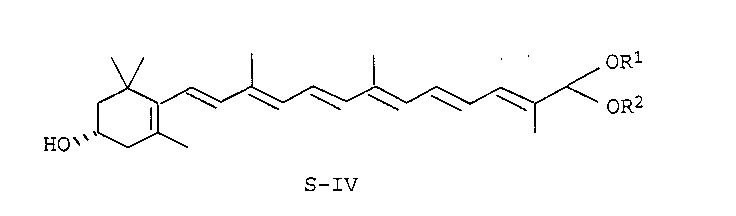
d) die C₂₅-Acetale R-IV oder S-IV in die C₂₅-Aldehyde R-V oder S-V überführt 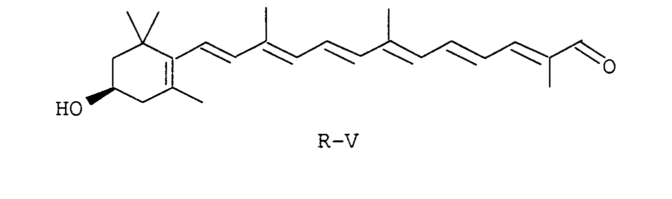 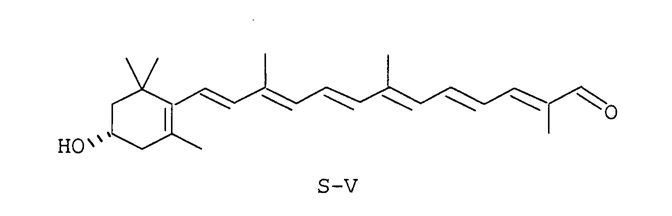
e) und den C₂₅-Aldehyd R-V mit dem C₁₅-Phosphoniumsalz S-II bzw. den C₂₅-Aldehyd S-V mit dem C₁₅-Phosphoniumalz R-II in einer Wittig-Reaktion zu sterisch einheitlichem meso-Zeaxanthin umsetzt.

Bei dem im Verfahrensschritt c) verwendeten G₁₀-Dial-monoacetal III kommen für offenkettige Acetale als Alkylreste R¹ und R² lineare oder verzweigte C₁-C₈-Alkylreste, z.B. Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl und n-Octyl in Frage.

Bevorzugte Alkylreste für R¹ und R² sind Methyl, Ethyl, n-Propyl und 1-Methylethyl, besonders bevorzugt Methyl und Ethyl.

Für cyclische Acetale kommen als Alkylreste für R³ bis R⁵ lineare oder verzweigte C₁-C₄-Alkylreste, z.B. Methyl, Ethyl, n-Propyl, 1-Methylethyl, n-Butyl, 1-Methylpropyl, 2-Methylpropyl und 1,1-Dimethylethyl in Frage.

Bevorzugte Reste für R³ bis R⁵ sind Wasserstoff und Methyl.

Der Rest Ph der C₁₅-Phosphoniumsalze R-II und S-II bezeichnet übliche, in Phosphinen und Phosphoniumsalzen vorkommende Arylreste, wie Phenyl, Toluol, Naphthyl, ggf. jeweils substituiert, bevorzugt Phenyl.

Der Rest X⁻ steht für ein Anionenäquivalent einer anorganischen oder organischen Säure, bevorzugt einer starken anorganischen oder organischen Säure.

Der Ausdruck starke Säure umfasst Halogenwasserstoffsäuren (insbesondere Salzsäure und Bromwasserstoffsäure), Schwefelsäure, Phosphorsäure, Sulfonsäuren und andere anorganische oder organische Säuren mit vergleichbarem Dissoziationsgrad. Als starke organische Säuren sind in diesem Zusammenhang auch C₁-C₆-Alkansäuren wie Ameisensäure, Essigsäure, Propionsäure, Buttersäure sowie Capronsäure zu verstehen.

Besonders bevorzugt sind Anionen solcher Säuren, ausgewählt aus der Gruppe, bestehend aus Salzsäure, Bromwasserstoffsäure, Schwefelsäure, Phosphorsäure, Ameisensäure, Essigsäure und Sulfonsäure zu nennen. Ganz besonders bevorzugt Cl⁻, Br⁻, CₙH₂ₙ₊₁-SO₃⁻ (mit n = 1-4), Ph-SO₃⁻, p-Tol-SO₃⁻ oder CF₃-SO₃⁻.

Zur Herstellung des racemischen Gemisches der Acetylendiole R-I und S-I geht man von Oxo-isophoron VIII aus, das in an sich bekannter Weise durch katalytische Hydrierung, beispielsweise mit Raney-Nickel in Methanol, in X überführt wird. Dabei wird als Zwischenstufe racemisches IX durchlaufen, das aber nicht isoliert werden muss. X fällt als trans/cis-Diastereomerengemisch an, wobei trans-X das überwiegende Hauptprodukt ist. Trans-X und cis-X liegen jeweils als Racemat vor. Die Trennung der Diastereomere kann nach einer in EP-A-0 775 685 diskutierten Methoden, vorzugsweise durch destillative Verfahren erfolgen.
Das dabei als Nebenprodukt anfallende racemische cis-X kann durch basenkatalysierte Epimerisierung an C₆ zu einem Gemisch aus racemischen cis-X und racemischen trans-X equilibriert und in die destillative Diastereomerentrennung rückgeführt werden. Das reine racemische trans-X wird gemäß der in Helv. Chim. Acta. 73 (4), 868, (1990) angegebenen Synthese in 3 Stufen in das racemische Gemisch R-I/S-I überführt.

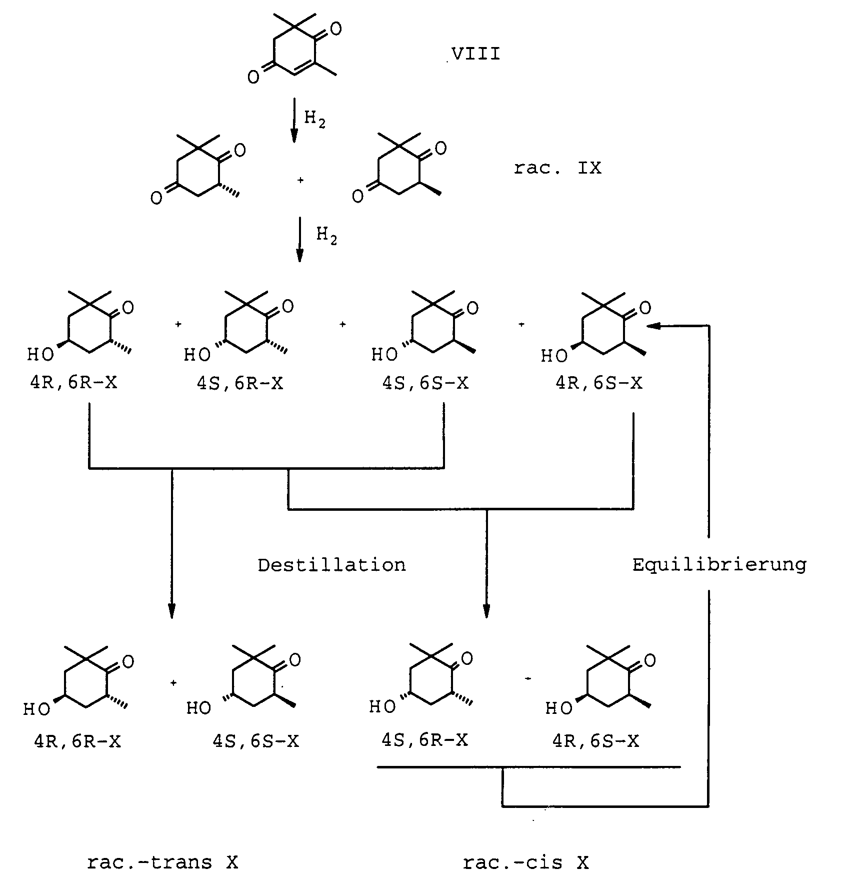

Das erfindungsgemäße Verfahren ist somit auch dadurch gekennzeichnet, dass es sich bei dem in der Stufe a) eingesetzten Gemisch um ein diastereomerenreines Racemat der Acetylendiole R-I und S-I handelt.

Die Auftrennung des racemischen Gemisches im Verfahrensschritt a) kann nach an sich bekannten Methoden erfolgen, beispielsweise durch enzymatisch katalysierte Enantiomerentrennung, durch Chromatographie an einer chiralen Säule oder durch Diastereomeren-Trennung.

Eine bevorzugte Variante des erfindungsgemäßen Verfahrens ist nun dadurch gekennzeichnet, dass man in der Stufe a) ein racemisches Gemisch der Acetylendiole R-I und S-I unter Verwendung eines optisch aktiven Hilfsreagenzes in ein Diastereomeren-Gemisch überführt, die Diastereomeren trennt und anschließend das Hilfsreagenz wieder abspaltet.

So wurde nun überraschenderweise gefunden, dass das racemische Gemisch der Acetylendiole R-I und S-I nach Derivatisierung mit optisch aktiven Hilfsreagenzien zu den diastereomeren Zwischenprodukten R-VI und S-VI, 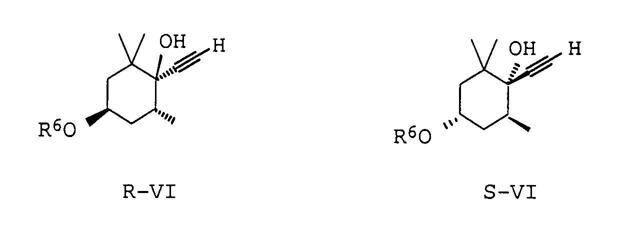 in denen der Substituent R⁶ bevorzugt einen optisch aktiven Urethan-, Carbonat-, Sulfonat- oder Acylrest bedeutet, in besonders einfacher Weise in seine Antipoden aufgetrennt werden kann.

Die Derivatisierung erfolgt völlig selektiv an der sekundären OH-Gruppe. Die Acetylendiole R-I und S-I erwiesen sich überraschenderweise sowohl chemisch als auch konfigurativ stabil gegenüber den Bedingungen, die für die Einführung der chiralen Hilfsgruppe, Trennung der diastereomeren Zwischenprodukte R-VI und S-VI und Abspaltung der Hilfsgruppe erforderlich sind.

Als diastereomere Zwischenprodukte R-VI und S-VI kommen prinzipiell alle Derivate in Betracht, über die racemische Alkohole in ihre Antipoden gespalten werden können (vgl. Houben-Weyl, Methoden der organischen Chemie, Alkohole, Teil III, S. 785 f., 1984).

Eine bevorzugte Ausführungsform des erfindungsgemäßen Verfahrens ist dadurch gekennzeichnet, dass man im Verfahrensschritt a) das Racemat selektiv an der sekundären OH-Gruppe mit einem optisch aktiven Hilfsreagenz, ausgewählt aus der Gruppe, bestehend aus Carbonsäuren, Carbonsäurehalogeniden, Chlorcarbonsäureestern, Sulfonsäuren und Isocyanaten derivatisiert.

Bei den diastereomeren Zwischenprodukten der Formeln R-VI und S-VI handelt es sich somit bevorzugt um Carbonsäureester, Sulfonsäureester, Carbonate und Urethane, aber auch um Monoester von Dicarbonsäuren, die ihrerseits mit optisch aktiven Aminen, beispielsweise mit Brucin, Ephedrin, Chinin, Menthylamin oder Strychnin in diastereomere Salze überführt werden können.

Zur Herstellung diastereomerer Urethane lässt sich beispielsweise ein racemisches Gemisch der Acetylendiole R-I und S-I mit Isocyanaten optisch aktiver Amine, wie z.B. (+)- oder (-)-Phenylethyl-isocyanat, (+)- oder (-)-1-(1-Naphthyl)-ethyl-isocyanat oder (+)- oder (-)-Menthyl-isocyanat in einem inerten Lösungsmittel umsetzen.

Carbonate werden beispielsweise durch Umsetzung von R-I bzw. S-I mit Estern der Chlorameisensäure, vorzugsweise mit Chlorameisensäure-menthylester, hergestellt.

Zur Herstellung diastereomerer Carbonsäure- bzw. Sulfonsäureester lässt sich beispielsweise das racemische Gemisch der Acetylendiole R-I und S-I mit ω-Camphansäure, Menthyloxy-essigsäure, Milchsäure, Mandelsäure, O,O-Diacetyl-weinsäure-methylester, α-Tosyl-aminocarbonsäuren, trans-Chrysanthemsäure, Campher-10-sulfonsäure oder mit deren Säurechloriden umsetzen.

Im Hinblick auf technische Realisierbarkeit sind diastereomere Ester besonders vorteilhaft, da das chirale Hilfsreagenz nach erfolgter Diastereomerentrennung und Esterverseifung durch einfache Säure/Basentrennung wiedergewonnen und in den Prozess zurückgeführt werden kann.

Als chirale Carbonsäuren sind zusätzlich zu den aus Houben-Weyl, Alkohole, Teil III, S. 785 f. (1984) bekannten und bereits oben genannten Verbindungen bevorzugt Derivate der D- oder L-Milchsäure wie z.B. α-Chlorpropionsäure, α-Phenoxypropionsäure sowie an der Phenylgruppe beliebig substituierte α-Phenoxypropionsäuren, besonders bevorzugt D- oder L-2,4-Dichlorphenoxypropionsäure, ganz besonders bevorzugt D-2,4-Dichlorphenoxypropionsäure oder D-2,4-Dichlorphenoxypropionsäurechlorid geeignet.

In einer bevorzugten Ausführungsform des erfindungsgemäßen Verfahrens wird das racemische Gemisch der Acetylendiole R-I und S-I in einem inerten Lösungsmittel in Gegenwart einer Base mit 1-1,2 Äquivalenten D-2,4-Dichlorpropionsäurechlorid bei etwa 0°C bis Raumtemperatur umgesetzt. Man erhält so in quantitativer Ausbeute ein Gemisch der diastereomeren 2,4-Dichlorphenoxypropionsäureester R-VIa und S-VIa.

Eine weitere vorteilhafte Ausführungsform des erfindungsgemäßen Verfahrens ist dadurch gekennzeichnet, dass man im Verfahrensschritt a) die diastereomeren Zwischenprodukte durch Kristallisation trennt.

So lässt sich beispielsweise durch Kristallisation aus dem rohen 1:1-Diastereomerengemisch der zur R-Reihe zugehörige diastereomere Ester R-VIa mit einer Reinheit > 95 Fp., bevorzugt > 97 % anreichern.

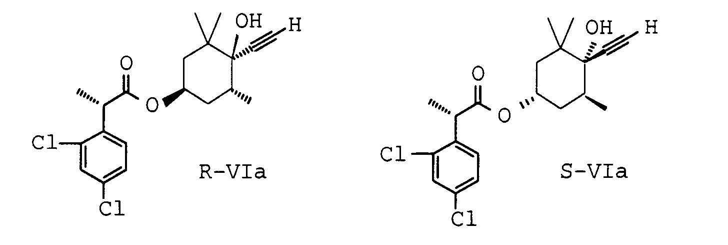

In der Mutterlauge wird der entsprechende diastereomere Ester S-VI angereichert. Besonders vorteilhaft gestaltet sich dieses Verfahren nun dadurch, dass nach der Verseifung der diastereomeren Ester in der Mutterlauge durch wiederholte Kristallisation das reine S-I mit einer Reinheit > 95 %, bevorzugt > 97 %, besonders bevorzugt > 99 % gewonnen werden kann.

Somit ist es möglich, mit einem Spaltreagens beide Enantiomeren in hoher Reinheit zu gewinnen. Außerdem kann durch geeignete Kristallisationen, Verseifung von Mutterlaugen und Re-Veresterungen das Racemat praktisch vollständig in die Enantiomeren gespalten werden. Durch diese Vorgehensweise ist es möglich, beide Enantiomeren in gleicher Menge zu erhalten, was für eine ökonomische Totalsynthese von meso-Zeaxanthin unabdingbar ist.

Nach der Racematspaltung können beide Enantiomeren, R-I und S-I, selektiv in das R-konfigurierte Phosphoniumsalz R-II bzw. das S-konfigurierte Phosphoniumsalz S-II überführt werden. Die Herstellung von R-II aus 1S,4R,6R-I (R-I) erfolgt dabei analog der in Helv. Chim. Acta 73 (4), 868 f. (1990) beschriebenen Synthese. Dieselbe Synthesesequenz ist in EP-A-0 283 979 für die Herstellung von 3R,3'R-Zeaxanthin offenbart.

Das erfindungsgemäße Verfahren zur Herstellung von meso-Zeaxanthin umfasst die Umsetzung des Acetylendiols S-I zum Phosphoniumsalz S-II. Dieser bislang noch nicht beschriebene Teilschritt wird analog zur Synthese von R-II - beschrieben in Helv. Chim. Acta 73 (4), 868 f. (1990) und EP-A-0 283 979 - durchgeführt.

Eine mögliche Synthesesequenz entspricht beispielsweise dem folgenden Reaktionsschema:

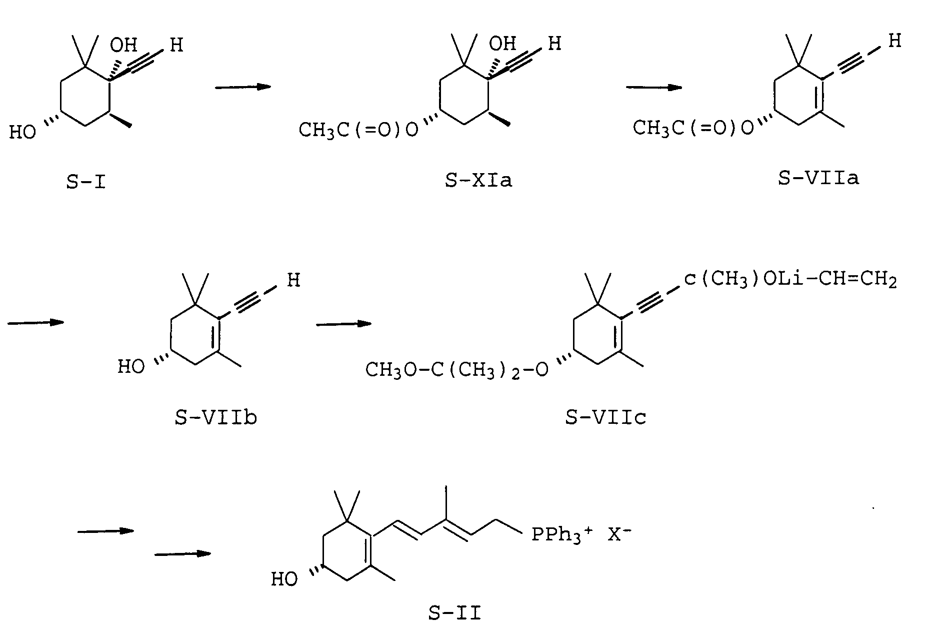

Neben der oben genannten Acetylschutzgruppe können selbstverständlich auch andere Acylreste wie z.B. Formyl- oder Propionylreste verwendet werden. Entsprechendes gilt für die Acetalschutzgruppe der Verbindung der Formel S-VIIc. Alternative Acetalschutzgruppen finden sich im späteren Teil der Beschreibung.

Einzelheiten zu den einzelnen Reaktionen finden sich in der oben zitierten Literatur.

Um aus den Phosphoniumsalzen R-II und S-II meso-Zeaxanthin zu erhalten, das völlig frei von R,R-Zeaxanthin und S,S-Zeaxanthin ist, müssen die Wittig-Reaktionen des zentralen C₁₀-Bausteins mit R-II bzw. S-II nacheinander vollständig selektiv ablaufen. Die für die Synthese eines einheitlichen Produkts erforderliche Selektivität ist nur dann gewährleistet, wenn ein C₁₀-Dialdehyd entsprechend der allgemeinen Formel III, eingesetzt wird, bei dem eine Carbonylgruppe als Acetal geschützt ist.

Für das erfindungsgemäße Verfahren wird vorzugsweise das Neopentylglycolacetal IIIa eingesetzt.

Die Umsetzung der Phosphoniumsalze R-II bzw. S-II mit IIIa über die Acetale R-IVa bzw. S-IVa zu den Aldehyden R-V bzw. S-V ist beschrieben in Helv. Chim. Acta 64 (7), 2489, 1981. Allerdings wurde auch hier nur im mmol-Maßstab gearbeitet. Die Aldehyde R-V und S-V wurden dort in aufwendiger Weise durch Kombination aus Chromatographie und Kristallisation isoliert. Die weitere Umsetzung zu meso-Zeaxanthin ist in dieser Publikation nicht beschrieben.

Eine weitere Aufgabe bestand somit darin, ein Verfahren zu finden, die Bausteine R-II, S-II und III in technisch realisierbarer Weise zu einheitlichem meso-Zeaxanthin zu verknüpfen. Überraschenderweise zeigte sich, dass man in hervorragende Ausbeute zu einem hochreinem meso-Zeaxanthin ohne Reinigung der anfallenden Zwischenprodukte gelangt.

Vorteilhafterweise geht man so vor, dass man R-II oder S-II (Reihenfolge beliebig) mit III, vorzugsweise mit IIIa unter den für Wittig-Reaktionen dieser Art beschriebenen Standardbedingungen (siehe Carotenoids, Vol. 2, "Synthesis", S. 79 ff.; Birkhäuser Verlag, 1996, und dort zitierte Literatur).umsetzt, wobei die Verwendung eines Oxirans als latente Base bevorzugt wird. Die rohen Acetale R-IV, bzw. S-IV können direkt sauer katalysiert zu den Aldehyden R-V bzw. S-V hydrolysiert werden. Prinzipiell sind hier alle Bedingungen zu sauer katalysierten Spaltung von Acetalen geeignet. Eine bevorzugte Ausführungsform der Acetalspaltung besteht aber darin, das Acetal in wässrigalkoholischem Medium mit katalytischen Mengen Zitronensäure (ca. 5 bis 50 mol-%, vorzugsweise 20 bis 30 mol-%) im Temperaturbereich von etwa 0°C bis Rückflusstemperatur, vorzugsweise bei 20 bis 30°C, zu verrühren.

Die Rohprodukte der Acetalspaltung, d.h. die rohen Aldehyde R-V bzw. S-V, werden mit den Phosphoniumsalzen S-II (für R-V) bzw. R-II (für S-V) unter den obengenannten Bedingungen der Wittig-Reaktion umgesetzt. Man erhält in hoher Ausbeute sterisch einheitliches meso-Zeaxanthin. Auch hier wird die Oxiran-Variante der Wittig-Reaktion bevorzugt, da man durch direkte Kristallisation aus dem Reaktionsgemisch zu einem Produkt hervorragender Reinheit gelangt.

Die Kondensation von R-II oder S-II mit III kann beispielsweise in einem inerten organischen Lösungsmittel z.B. in offenkettigen oder cyclischen Ethern wie Diethylether, Diisopropylether, Methyl-tert.butylether, 1,4-Dioxan oder THF, in halogenierten Kohlenwasserstoffen wie Dichlormethan, Chloroform, in aromatischen Kohlenwasserstoffen wie Toluol, Xylol oder Benzol oder in polaren Lösungsmitteln wie Dimethylformamid, Dimethylsulfoxid oder Acetonitril durchgeführt werden.

Als Base können alle für Wittig-Kondensationen üblichen Basen verwendet werden, z.B. Alkalimetallhydroxide wie Natriumhydroxid, Kaliumhydroxid oder Lithiumhydroxid; Alkalimetallhydride wie Natriumhydrid oder Kaliumhydrid.

Als Basen kommen außerdem Lithiumorganyle wie z.B. n-Butyllithium, tert. Butyllithium, Phenyllithium oder Alkalimetallamide wie Lithium-, Kalium- oder Natriumamid, Lithium-diisopropylamid aber auch Alkalimetallhexamethyldisilazide in Frage.

Die Menge an eingesetzter Base liegt in der Regel im Bereich von 0,8 bis 5 Mol, bevorzugt 1 bis 3 Mol pro Mol des eingesetzten Phosphoniumsalzes II.

Wenn X⁻ ein Halogenidanion ist, können auch Oxirane vorteilhaft als latente Basen eingesetzt werden (siehe Chem. Ber. 1974, 107, 2050).

Vorzugsweise werden für diese Wittig-Reaktion als Basen Lösungen von Alkalimetallalkoholaten im korrespondierenden Alkohol oder Oxirane, vor allem 1,2-Epoxibutan, ohne zusätzliches Solvens oder im Gemisch mit einem der obengenannten Lösungsmittel oder einem niederen Alkanol verwendet.

Somit konnte die Aufgabe, aus den Phosphoniumsalzen R-II und S-II zu sterisch einheitlichem meso-Zeaxanthin hoher chemischer Reinheit zu gelangen, ohne Reinigung von Zwischenprodukten in technisch sinnvoller Weise gelöst werden.

Gegenstand der Erfindung ist ebenfalls ein Verfahren zur Herstellung von optisch reinen Acetylendiolen der Formeln R-I und S-I, 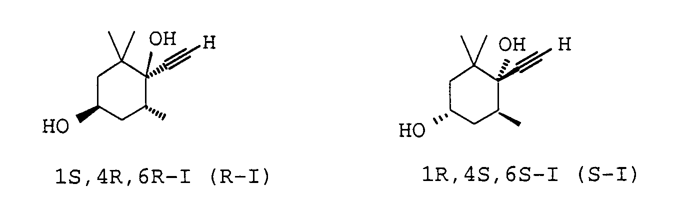 dadurch gekennzeichnet, dass man ein racemisches Gemisch der Acetylendiole R-I und S-I unter Verwendung eines optisch aktiven Hilfsreagenzes in ein Diastereomeren-Gemisch überführt und dieses in seine Antipoden auftrennt.

Das Verfahren ist dadurch gekennzeichnet, dass es sich bei dem Gemisch um ein diastereomerenreines Racemat handelt.

Das Verfahren ist ferner dadurch gekennzeichnet, dass man das Racemat selektiv an der sekundären OH-Gruppe mit einem optisch aktiven Hilfsreagenz, ausgewählt aus der Gruppe, bestehend aus Carbonsäuren, Carbonsäurehalogeniden, Chlorcarbonsäureestern, Sulfonsäuren und Isocyanaten zu einem Gemisch diastereomerer Zwischenprodukte der Formeln R-VI und S-VI derivatisiert, 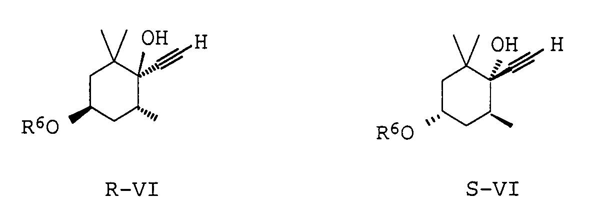 in denen der Substituent R⁶ einen optisch aktiven Urethan-, Carbonat-, Sulfonat- oder einen Acylrest bedeutet.

Als optisch aktives Hilfsreagenz werden bevorzugt D- oder L-Milchsäure-Derivate, besonders bevorzugt D-2,4-Dichlorphenoxypropionsäure oder D-2,4-Dichlorphenoxypropionsäurechlorid eingesetzt.

Eine vorteilhafte Ausführungsform des Verfahrens ist dadurch gekennzeichnet, dass man die diastereomeren Zwischenprodukte durch Kristallisation trennt.

Gegenstand der Erfindung sind auch optisch aktive Cyclohexanderivate der allgemeinen Formeln R-VI und S-VI, 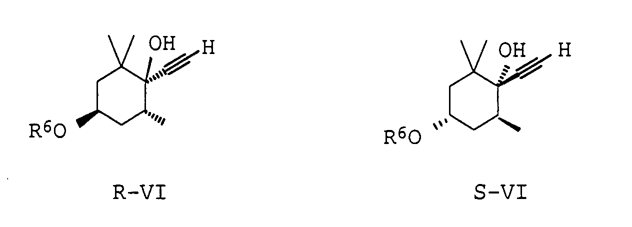 in denen der Substituent R⁶ ein optisch aktiven Urethan-, Carbonat-, Sulfonat- oder ein Acylrest bedeutet.

Gegenstand der Erfindung sind auch 2,4-Dichlorphenoxypropionsäureester der Formeln R-VIa und S-VIa sowie R-VIb und S-VIb

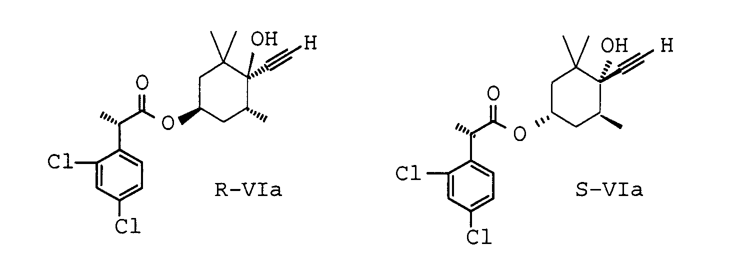

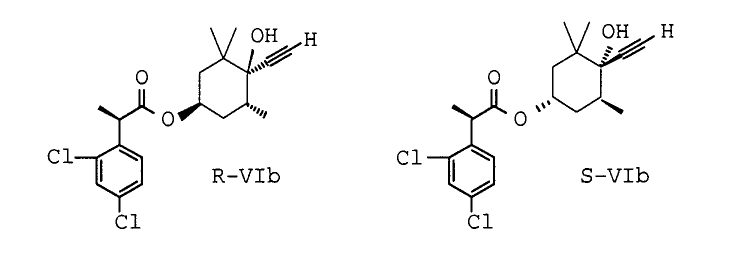

Gegenstand der Erfindung ist auch ein optisch aktives Acetylendiol der Formel S-I

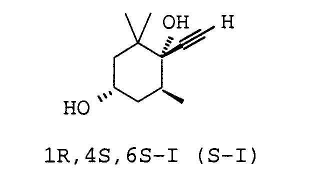

Gegenstand der Erfindung sind auch optisch aktive Acetylenverbindungen der allgemeinen Formel S-VII, 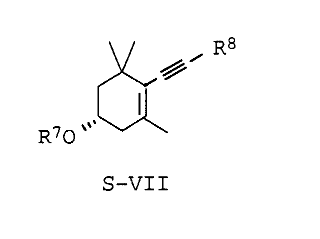 in denen die Substituenten unabhängig voneinander folgende Bedeutung haben:
- R⁷: Wasserstoff, C₁-C₁₂-Acyl oder eine hydrolytisch spaltbare Acetal oder Etherschutzgruppe;
- R8: Wasserstoff oder C(CH₃)OR⁹-CH=CH₂;
- R9: Lithium oder Wasserstoff.

Als Acylreste für R⁷ sind verzweigte oder unverzweigte, gesättigte oder ungesättigte C₁-C₁₂-Acylreste zu verstehen.

Beispiele hierfür sind Acylreste der Ameisen-, Essig-, Propion-, n-Butter-, iso-Butter-, Sorbin-, n-Valerian-, iso-Valerian-, Capron-, Capryl-, Caprin-, Undecan- und Laurinsäure. Bevorzugt sind Acylreste der Ameisen-, Essig- und Propionsäure, besonders bevorzugt Acetat.

Unter hydrolytisch spaltbare Acetal oder Etherschutzgruppen für R⁹ sind durch Hydrolyse in eine Hydroxygruppe überführbare Schutzgruppen zu verstehen. Genannt seien beispielsweise Ethergruppen, wie 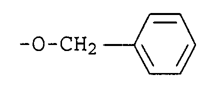 und -O-C(CH₃)₃
Silylethergruppen, wie -O-Si(CH₃)₃, -O-Si(CH₂CH₃)₃, -O-Si(iso-Propyl)₃, -O-Si(CH₂CH₂)₂(i-Propyl), -O-Si(CH₃)₂(tert.-Butyl) und -O-Si(CH₃)₂(n-Hexyl) oder substituierte Methylethergruppen, wie die α-Alkoxy- oder α-Aryloxy-alkylethergruppen der Formeln 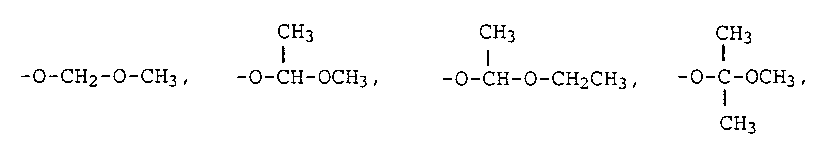 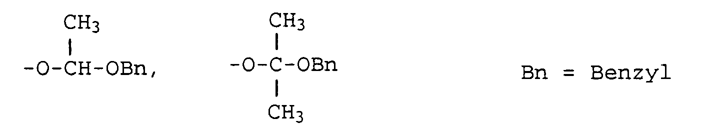 und geeignete Pyranylethergruppen, wie die Tetrahydropyranyloxygruppe und die 4-Methyl-5,6-dihydro-2H-pyranyloxy-Gruppe.

Bevorzugt verwendet man für R³ die Tetrahydropyranyloxygruppe 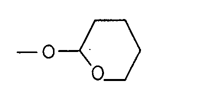 oder die α-Ethoxy-ethoxygruppe der Formel

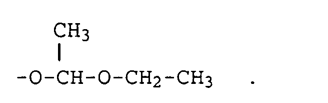

Die entsprechenden Reaktionsbedingungen zur Einführung und Abspaltung der o.g. Schutzgruppen finden sich u.a. in T. Greene "Protective Groups in Organic Chemistry", John Wiley & Sons, 1981, Chapter 2.

Gegenstand der Erfindung sind ebenso optisch aktive Cyclohexanderivate der allgemeinen Formel S-XI, 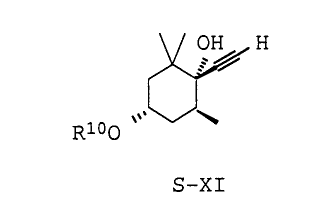 in denen R¹⁰ einen nicht-chiralen C₁-C₁₂-Acyl oder eine hydrolytisch spaltbare Acetal- oder Etherschutzgruppe bedeutet. Die nähere Definition der Reste R¹⁰ - allgemein und in der bevorzugten Ausführungsform - entsprechen der oben genannten Beschreibung für R⁷.

Anhand der folgenden Beispiele soll das erfindungsgemäße Verfahren näher erläutert werden.

### Beispiel 1a

### Racemisches 1S,4R,6R-I/1R,4S,6S-I

156,4 g (1,0 mol) racemisches-trans-X (Reinheit nach GC: 99,9 %) wurden in 250 ml THF gelöst. Man gab 0,25 g (1 mmol) Pyridinium-4-toluolsulfonat zu und tropfte dann innerhalb von 45 min. 163,2 g (2,15 mol) Isopropenyl-methylether (Reinheit nach GC: 95 %) zu. Die Reaktionstemperatur wurde dabei durch Wasserbadkühlung bei etwa 25°C gehalten. Nach Beendigung des Isopropenylmethyl-ether-Zulaufs wurde noch 2h bei 25°C nachgerührt.

Das Reaktionsgemisch wurde anschließend innerhalb von 1h bei 0°C zu einer Suspension von Lithiumacetylid in THF gegeben.

Die Lithiumacetylid-Suspension wurde folgendermaßen hergestellt:

Zu 750 ml flüssigem Ammoniak wurden innerhalb von 1 Stunde bei -40°C portionsweise insgesamt 14,0 g (2,0 mol) Lithium-Granulat zugegeben. Anschließend wurde bei -40°C innerhalb von 3h mit 150 1 Acetylen begast. Bei -40°C gab man 750 ml THF zu und ließ dann die Temperatur innerhalb von 90 min langsam auf 0°C steigen, wobei weiterhin mit Acetylen (50 l/h) begast wurde.

Nach Zugabe der Acetalisierungslösung von rac-trans-X wurde 1 Stunde bei 0°C nachgerührt. Anschließend tropfte man innerhalb von 1 Stunde bei 0°C 400 ml Eiswasser zu. Man ließ auf Raumtemperatur kommen. Nach Zugaben von 700 ml Hexan wurde die wässrige Unterphase abgetrennt und zweimal mit je 700 ml Hexan nachextrahiert. Die vereinigten organischen Phasen wurden einmal mit jeweils 700 ml halbkonzentrierter Ammoniumchloridlösung und halbkonzentrierter Kochsalzlösung gewaschen, über Natriumsulfat getrocknet und am Rotationsverdampfer eingeengt. Der Rückstand (287 g, hellgelber Festkörper) wurde in 1300 ml THF gelöst. Man gab 52 ml Wasser und 2,51 g Pyridinium-4-toluolsulfonat zu und rührte 1 Stunde bei Raumtemperatur nach. Dann verdünnte man mit 700 ml Ethylacetat und wusch mit 500 ml gesättigter AmmoniumchloridLösung. Die organische Phase wurde anschließend mit 500 ml gesättigter Kochsalzlösung gewaschen. Die vereinigten Wasserphasen wurden zweimal mit je 250 ml Ethylacetat nachextrahiert. Die vereinigten organischen Phasen wurden über Natriumsulfat getrocknet und am Rotationsverdampfer eingeengt. Der Rückstand wurde aus einem Gemisch aus 250 ml Ethylacetat und 750 ml Diisopropylether kristallisiert. Man erhielt 124 g Erstkristallisat mit einer Ausbeute von 68,1 %, bezogen auf rac.-trans-X. Reinheit nach GC: 98,9 %; Fp: 124,5 bis 125°C.

Das Filtrat wurde auf ca. 300 ml eingeengt. Man rührte 1 h bei 0°C aus und filtriert das Zweitkristallisat ab. Auswaage Zweitkristallisat: 23 g; Ausbeute: 12,6 % bezogen auf rac.-trans-X; Reinheit nach GC: 98,4 %; Fp: 124 bis 124,5°C.

### Beispiel 1b

### Überführung von 1S,4R,6R-I/1R,4S,6S-I in die diastereomeren D-2,4-Dichlorphenoxypropionsäureester R-VIa und S-VIa

182 g (1,0 mol) kristallines R-I/S-I wurden in einem Gemisch aus 95 g (1,1 mol) Pyridin und 2000 ml Methyl-tert.butylether gelöst. Innerhalb von 1 h wurden bei 0 bis 5°C 290 g (1,1 mol) D-2,4-Dichlorphenoxy-propionsäurechlorid (Reinheit nach GC: 96,2 %) zudosiert und der Reaktionsansatz 1 Stunde bei dieser Temperatur nachgerührt. Nach anschließender Zugabe von 300 ml Wasser wurde die wässrige Unterphase abgetrennt. Die organische Oberphase wurde einmal mit je 150 ml 5%iger Schwefelsäure, Wasser, gesättigter Bicarbonatlösung und nochmals Wasser gewaschen, über Natriumsulfat getrocknet und am Rotationsverdampfer eingeengt.

Bei vier gleichartigen Ansätzen wurden folgende Auswaagen erhalten:

| Ansatz | Auswaage diastereomere Ester | GC Gehalt (Fl.-%) |
|---|---|---|
| 1 | 421.3 g | 97,9 % |
| 2 | 418.3 g | 98,4 % |
| 3 | 425.3 g | 98,3 % |
| 4 | 424,0 g | 98,8 % |

### Beispiel 1c

### Diastereomerentrennung

In zwei Ansätzen wurden je 840 g der in Beispiel 1b erhaltenen diastereomeren Ester aus einer Mischung aus 2000 ml Hexan und 200 ml Diisopropylether kristallisiert. Die Kristallisate beider Ansätze und die Mutterlaugen beider Ansätze wurden vereinigt.

Die Gesamtauswaage an Kristallisat betrug 575,2 g, die Menge an Mutterlaugeneindampfrückstand betrug 1142 g.

Zusammensetzung nach GC-Analyse (Fl.-%):

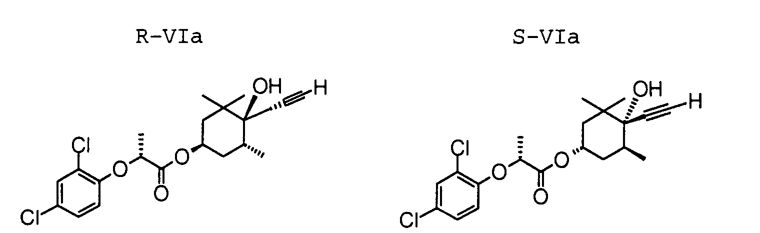

| | | |
|---|---|---|
| Kristallisat | 76 % | 24 % |
| Mutterlaugenrückstand | 32 % | 60 % |

### Beispiel 1d

### Hochreinigung von R-VIa

Das Kristallisat aus Beispiel 1c (575 g) wurde bei 50°C in 350 ml Diisopropylether gelöst. Nach Zugabe von 1725 ml n-Hexan wurde der Reaktionsansatz auf Raumtemperatur abgekühlt und über Nacht gerührt. Das Kristallisat wurde abfiltriert und im Stickstoffstrom getrocknet. Auswaage Kristallisat: 372 g; Zusammensetzung nach GC: 89,9 % R-VIa, 10,4 % S-VIa.

Das Filtrat wurde am Rotationsverdampfer eingeengt. Auswaage Mutterlaugenrückstand: 190,9 g; Zusammensetzung nach GC: 42,6 % R-VIa, 56,5 % S-VIa.

Das Kristallisat (372 g) wurde nochmals aus 225 ml Diisopropylether und 1120 ml n-Hexan umkristallisiert. Auswaage: 305,2 g; Fp: 84,5 bis 85°C, Reinheit nach GC: > 97 % R-VIa; Drehwert (D 25°C): +9,74° (c = 1 in Methyltert.butylether).

Das Filtrat wurde am Rotationsverdampfer eingeengt. Auswaage Mutterlaugenrückstand: 67,2 g; Zusammensetzung nach GC: 59,2 % R-VIa, 40,8 % S-VIa.

Die vereinigten Mutterlaugenrückstände (258 g) enthielten die diastereomeren Ester R-VIa und S-VIa annähernd im Verhältnis 1:1 und daneben keine anderen Nebenkomponenten. Sie konnten direkt in die Diastereomerentrennung gemäß Beispiel 1c rückgeführt werden.

### Beispiel 1e

### Verseifung des diastereomeren Esters R-VIa

300,0 g (0,75 mol) der Verbindung R-VIa aus Beispiel 1d wurden in 1,5 1 Methanol gelöst. Man ließ bei Raumtemperatur eine Lösung von 110 g Kaliumhydroxid in 1,5 1 Methanol zulaufen und rührte über Nacht bei Raumtemperatur nach. Anschließend wurden 990 g 10%ige Essigsäurelösung zugesetzt und das Reaktionsgemisch nach wenigen Minuten Nachrührzeit am Rotationsverdampfer aufkonzentriert. Zum Rückstand gab man 0,5 1 gesättigte Kochsalzlösung und extrahierte das Gemisch dann einmal mit 750 ml und dreimal mit je 250 ml Methyl-tert.butylether. Die vereinigten organischen Phasen wurden dreimal mit je 400 ml gesättigter Bicarbonatlösung und einmal mit 200 ml Wasser gewaschen, über Natriumsulfat getrocknet und am Rotationsverdampfer eingeengt.

Der Eindampfrückstand wurde in 200 ml Ethylacetat in der Hitze gelöst. Man gab 450 ml Diisopropylether zu und ließ dann Reaktionsansatz über Nacht bei 5°C stehen. Das Kristallisat wird abfiltriert und im Stickstoffstrom getrocknet. Auswaage: 116,9 g, R-I; Ausbeute: 85,6 % d.Th.; Fp: 150 bis 150,5°C; Reinheit nach GC: 98,5 % R-I (25 m Chirasil-Dex.); Drehwert (D, 25°C): -28,39° (C=1 in Methanol).

Das Kristallisat wurde gemäß den Angaben in der Literatur (Helv. Chim. Acta 73, 868(1990) sowie EP 283979 in das R-konfigurierte C₁₅-Phosphoniumsalz R-II überführt.

### Beispiel 1f

### Verseifung des an diastereomerem Ester S-VIa angereicherten Mutterlaugenrückstandes aus Beispiel 1c

Der Mutterlaugenrückstand aus Beispiel 1c wurde in zwei gleichen Ansätzen wie folgt weiterverarbeitet: Jeweils 533 g (1,335 mol) wurden in 2,7 1 Methanol gelöst und bei Raumtemperatur mit einer Lösung von 194 g Kaliumhydroxid in 2,7 1 Methanol versetzt. Nach mehrstündigem Rühren gab man 1,77 kg 10%ige Essigsäure hinzu und rührte kurz nach. Beide Ansätze wurden vereinigt und am Rotationsverdampfer bei 50°C Badtemperatur aufkonzentriert. Der Rückstand wurde mit 1870 ml gesättigter Kochsalzlösung versetzt und einmal mit 2,7 1 sowie dreimal mit je 800 ml Methyl-tert.butylether extrahiert. Die vereinigen organischen Phasen wurden fünfmal mit je 1,3 1 gesättigter Bicarbonatlösung und einmal mit 700 ml Wasser gewaschen, über Natriumsulfat getrocknet und am Rotationsverdampfer eingeengt. Auswaage: 355 g; GC-Analyse (25 m Chirasil-Dex): 57,8 % (1R,4S,6S)-I (S-I), 34,7 % (1S,4R,6R)-I (R-I).

Die vereinigten Wasserphasen wurden am Rotationsverdampfer auf etwa das halbe Volumen aufkonzentriert. Der entstandene Niederschlag wurde abfiltriert und mit 2 1 Methyl-tert. butylether und 1,5 1 Wasser aufgenommen. Die organische Phase wurde mit 500 ml Wasser gewaschen. Die beiden wässrigen Phasen wurden vereinigt und zweimal mit je 500 ml Methyl-tert.-Butylether reextrahiert. Die vereinigten organischen Phasen wurden über Natriumsulfat getrocknet und am Rotationsverdampfer eingeengt.

Beide Eindampfrückstände wurden für die Hochreinigung (s. Beispiel Ii) vereinigt. Auswaage: 63,4 g; GC-Analyse (25 m Chirasil-Dex): 82,7 % S-I, 17,2 % R-I.

### Beispiel 1g

### Rückgewinnung der D-2,4-Dichlorphenoxypropionsäure

Die vereinigten Wasserphasen aus Beispiel 1f wurden am Rotationsverdampfer auf ca. die Hälfte des ursprünglichen Volumens aufkonzentriert. Das Konzentrat wurde durch Zutropfen von 97%iger Schwefelsäure bei Raumtemperatur auf pH 1 gestellt. Man rührte 30 min. nach und filtrierte ab. Der Filterkuchen wurde dreimal mit je 11 Wasser gewaschen und bei 50°C/10 mbar getrocknet. Auswaage: 565 g; Ausbeute: 90,0 %; Fp: 110 bis 115°C; Reinheit nach GC: 97,8 %.

Zur Hochreinigung wurde dieses Material aus 31 Toluol umkristallisiert. Fp: 122 bis 122,5°C; Drehwert (D, 25°C): +40,02° (C=1 in Methyl-tert.butylether).

### Beispiel 1h

### Herstellung von D-2,4-Dichlorphenoxypropionsäurechlorid

235 g (1,0 mol) D-2,4-Dichlorphenoxypropionsäure wurden vorgelegt. Man ließ 179 g (1,5 mol) Thionylchlorid zulaufen und erwärmte im Verlauf von 3 h langsam auf 100°C. Man rührte 1 h bei 100°C nach und zog dann bei 100°C/20 mbar überschüssiges Thionylchlorid ab. Der Rückstand wurde über eine Destillationsbrücke gereinigt. Bei 130°C/6 mbar gingen 245,8 g D-2,4-Dichlorphenoxypropionsäurechlorid über. Ausbeute: 96,7 % d.Th.; Reinheit nach GC: 100 %; Drehwert (D, 25°C): +33,23° (C=1 in n-Hexan).

### Beispiel 1i

### Hochreinigung von S-I

417 g vereinigter Eindampfrückstand aus Beispiel 1f wurden in der Hitze in 730 ml Ethylacetat gelöst. Man gab 1460 ml Diisopropylether zu und führte eine Heißfiltration durch. Man ließ auf Raumtemperatur kommen und rührte über Nacht nach. Das Kristallisat wurde abgesaugt und im Stickstoffstrom getrocknet. Auswaage Kristallisat 1: 246,6 g; GC-Analyse (25 m Chirasil-Dex): 72,8 % S-I, 25,3 % R-I.

Das Filtrat wurde am Rotationsverdampfer eingeengt. Auswaage Mutterlauge 1: 167,9 g; GC-Analyse (25 m Chirasil-Dex): 43,5 % S-I, 46,8 % R-I.

Das Kristallisat 1 wurde in der Hitze in 450 ml Ethylacetat aufgenommen und nach Zugabe von 900 ml Diisopropylether wie oben kristallisiert. Auswaage Kristallisat 2: 175,9 g; GC-Analyse (25 m Chirasil-Dex): 86,9 % S-I, 12,8 % R-I.

Das Filtrat wurde am Rotationsverdampfer eingeengt. Auswaage Mutterlauge 2: 70,1 g; GC-Analyse (25 m Chirasil-Dex): 48,3 % S-I, 51, 3 % R-I.

Das Kristallisat 2 wurde wie oben beschrieben, nochmal aus 300 ml Ethylester und 630 ml Diisopropylether umkristallisiert. Dabei erhielt man als Kristallisat 3 ein optisch reines 1R,4S,6S-I. Auswaage Kristallisat 3: 130,8 g; Fp: 150,5 bis 151°C; GC-Analyse (25 m Chirasil-Dex): 99,3 % S-I (das Enantiomer R-I wurde nicht detektiert); Drehwert (D, 25°C): +28,32° (C=1 in Methanol).

Das Filtrat wurde am Rotationsverdampfer eingeengt. Auswaage Mutterlauge 3: 44,9 g; GC-Analyse (25 m Chirasil-Dex): 53,6 % S-I, 45,8 % R-I.

Die Mutterlaugenrückstände 1 bis 3 wurden vereinigt (282,9 g). Sie enthielten die beiden Enantiomeren S-I und R-I annähernd im Verhältnis 1:1. Sie konnten direkt in die Derivatisierung zu den diastereomeren D-2,4-Dichlorphenoxypropionsäureestern gemäß Beispiel 1b rückgeführt werden.

### Beispiel 1j

### (1R,4S,6S)-C₁₁-Mono-Acetat S-XIa

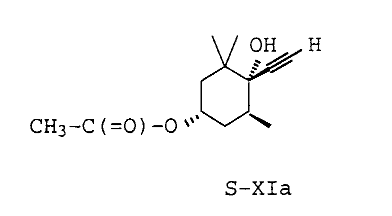

242,4 g (1,33 mol) S-I wurden in 463 g (5,85 mol) Pyridin gelöst. Man kühlte auf 0°C ab und ließ innerhalb von 30 min 543 g (5,32 mol) Acetanhydrid zulaufen. Man ließ auf Raumtemperatur kommen und rührte dann 4 h bei 50°C nach.

Zur Aufarbeitung wurde das Reaktionsgemisch am Rotationsverdampfer eingeengt. Der Rückstand wurde in 1,3 1 Methylenchlorid gelöst. Die resultierende Lösung wurde je einmal mit 650 ml 5%iger Salzsäure, 330 ml gesättigter Kochsalzlösung und 330 ml gesättigter Bicarbonatlösung gewaschen. Die vereinigten wässrigen Phasen wurden zweimal mit je 130 ml Methylenchlorid reextrahiert. Die vereinigten organischen Phasen wurden über Natriumsulfat getrocknet und am Rotationsverdampfer eingeengt. Der Rückstand (334 g) wurde mit 3 1 Hexan aufgenommen und bei ca. 50°C mit 15 g Aktivkohle behandelt. Nach Klärfiltration ließ man auf Umgebungstemperatur abkühlen und rührte anschließend 1 h im Eiswasserbad nach. Das Kristallisat wurde abgesaugt, mit kaltem n-Hexan gewaschen und im Stickstoffstrom getrocknet. Auswaage: 246,1 g; Ausbeute: 82,6 % d.Th.; Fp: 69°C; Reinheit nach GC: 100 %; Drehwert (D, 25°C): +23,14° (C=1 in EtOH).

Die Mutterlauge wurde am Rotationsverdampfer eingeengt. Durch Kristallisation des Eindampfrückstands (49,7 g) aus 200 ml n-Hexan erhielt man folgendes Zweitkristallisat: Auswaage: 40,3 g; Ausbeute: 13,5 % d.Th.; Fp: 68 bis 68,5°C; Reinheit nach GC: 99,44%; Drehwert (D 25°C): +23,09° (C=1 in EtOH). Gesamtauswaage: 286,4 g; Gesamtausbeute: 96,1 % d.Th.

### Beispiel 1k

### C₁₁-Acetat S-VIIa

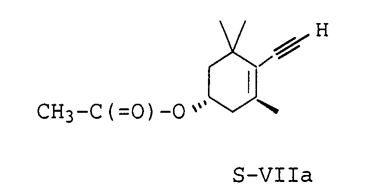

142,4 g (635 mmol) S-XIa wurden in 1,3 1 Ortho-Xylol gelöst. Man gab 15,89 g (63,5 mmol) Kupfer-(II)-sulfat-Pentahydrat zu, erhitzte auf Rückfluss und kreiste 2 h Wasser aus. Dann ließ man auf 90°C abkühlen, gab nochmals 15,85 g (63,5 mmol) Kupfer-(II)-sulfat-Pentahydrat zu und kreiste nochmals 4 h Wasser aus.

Zwei gleichartige Ansätze wurden vereint und über Celite klärfiltriert. Man wusch zweimal mit je 500 ml Hexan nach. Die vereinigten Filtrate wurden zweimal mit je 500 ml Wasser und zweimal mit 500 ml halbkonzentrierter Bicarbonat-Lösung gewaschen, über Natriumsulfat getrocknet und am Rotationsverdampfer eingeengt. Der Rückstand (342 g) wurde durch Vakuum-Destillation über eine 10 cm-Vigreux-Kolonne gereinigt. Bei 9,5 mbar/105-107°C Übergangstemperatur destillierten 236,4 g (90,4 % d.Th.) S-VIIa mit einer Reinheit von 99,1 % nach GC über. Drehwert (D, 25°C): +68,04° (C=1 in EtOH).

### Beispiel 1l

### C₁₁-Acetylenalkohol S-VIIb

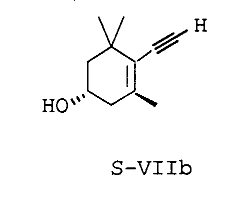

235,6 g (1,14 mol) S-VIIa wurden in 1,1 1 Methanol gelöst. Innerhalb von 30 min. wurden bei 5°C bis 10°C portionsweise 93,2 g Kaliumhydroxid eingetragen. Man ließ auf Raumtemperatur kommen und rührte 30 min. nach. Dann schüttete man das Reaktionsgemisch auf ein Gemisch aus 2,3 1 Wasser und 34 g Essigsäure und extrahierte dreimal mit je 600 ml Methylenchlorid. Die vereinigten organischen Phasen wurden je einmal mit 600 ml gesättigter Kochsalzlösung und 700 ml gesättigter Bicarbonatlösung gewaschen, über Natriumsulfat getrocknet und am Rotationsverdampfer eingeengt. Der Rückstand (189 g) wurde in der Hitze in 1 l n-Hexan aufgenommen und mit 5 g Aktivkohle entfärbt. Nach Abtrennung der Aktivkohle kühlte man auf 5°C ab und rührte 1 h im Eiswasserbad aus. Das Kristallisat wurde abfiltriert und im Stickstoffstrom trockengeblasen. Auswaage: 176,3 g; Ausbeute: 94,3 % d.Th.; Fp: 75 bis 75, 5°C; Drehwert (D, 25°C): +129° (C=1 in Ethanol); Reinheit nach GC: 99,94 %.

Aus dem Mutterlaugenrückstand (11 g) wurden durch Kristallisation aus n-Hexan 5,5 g (2,9 % d.Th.) Zweitkristallisat gewonnen. Fp: 70 bis 71°C; Drehwert (D, 25°C): +121,2° (C=1 in Ethanol); Reinheit nach GC: 94,93 %.

### Beispiel 1m

### C₁₅-Phosphoniumsalz S-II

90,33 g (0,55 mol) C₁₁-Acetylenalkohol S-VIIb wurden in 550 ml THF gelöst. Man gab 1,38 g (5,5 mmol) Pyridiniumtosylat zu. Innerhalb von 30 min. tropfte man bei 20°C 99,2 g (1,38 mol) frisch destillierten Isopropenylmethylether zu. Man rührte 1 h bei Umgebungstemperatur nach.

Die so erhaltene Lösung des Acetals wurde dann auf -20°C abgekühlt. Innerhalb von 30 min. ließ man bei -10°C bis -15°C 282 g einer 15%igen Lösung von n-Butyllithium in Hexan zulaufen (= 0,66 mol n-Butyllithium). Man rührte 15 min. bei -15°C nach und tropfte dann innerhalb von 30 min bei -10°C eine Lösung von 47,76 g (0,55 mol) Lithiumbromid in 410 ml THF zu. Man rührte 10 min. bei -10°C nach und gab dann bei -10°C innerhalb von 30 min. 57,8 g (0,825 mol) frisch destilliertes 1-Buten-2-on zu. Man rührte 45 min. bei -10°C nach. Dann wurden bei dieser Temperatur innerhalb von 30 min. 191 g einer 70%igen toluolischen Lösung von Natrium-dihydro-bis-(2-methoxy-ethoxy)-aluminat zugetropft. Nach Zutropfende wurde 10 min. bei -10°C nachgerührt. Man ließ auf 0°C aufwärmen und rührte 1 h bei 0°C nach.

Zur Aufarbeitung ließ man bei 0°C ein Gemisch aus n-Hexan/Ethanol [80/40 (v/v)] zulaufen. Anschließend gab man bei 0°C 825 ml 28%ige Natronlauge zu. Man rührte 15 min. bei 0°C nach und trennte dann die organische Oberphase ab. Die wässrige Unterphase wurde dreimal mit je 500 ml n-Hexan nachextrahiert. Die vereinigten organischen Phasen wurden mit 350 ml gesättigter Kochsalzlösung gewaschen, über Natriumsulfat getrocknet und am Rotationsverdampfer eingeengt. Der Rückstand wurde bei 50°C getrocknet.

Aus zwei gleichartigen Ansätzen erhielt man folgende Auswaagen: Ansatz A: 170 g

### Ansatz B: 173 g

349,7 g (1,32 mol) Triphenylphosphin wurden in 1,1 1 Methanol suspendiert. Bei 0°C tropfte man 137 g 37%ige Salzsäure zu und rührte 15 min. bei 0°C nach. Dazu wurde bei 0°C in 4 h eine Lösung der vereinigten Eindampfrückstände aus Ansatz A und Ansatz B (343 g) in 350 ml Methanol getropft. Das Gemisch wurde über Nacht bei Raumtemperatur nachgerührt.

Zur Aufarbeitung versetzte man mit 550 ml Wasser und 1,3 1 n-Hexan. Die wässrige Unterphase wurde abgetrennt und dreimal mit je 1,3 1 n-Hexan gewaschen. Die vereinigten Hexanphasen wurden verworfen. Die wässrige Unterphase wurde mit 900 ml Wasser verdünnt und anschließend 30 min. bei Raumtemperatur mit 45 g Aktivkohle verrührt. Die Aktivkohle wurde abfiltriert und mit 200 ml Wasser nachgewaschen. Das Filtrat wurde am Rotationsverdampfer auf ein Gesamtvolumen von ca. 1,8 1 aufkonzentriert. Das Konzentrat wurde dreimal mit je 1,5 1 Methylenchlorid extrahiert; die vereinigten organischen Phasen wurden zweimal mit je 700 ml gesättigter Kochsalzlösung gewaschen, über Natriumsulfat getrocknet und am Rotationsverdampfer eingeengt. Der Rückstand wurde in der Hitze in 1,1 1 Acetonitril gelöst. Nach Zugabe von 2,2 1 Ethylacetat wurde auf 5°C abgekühlt. Das Kristallisat wird abfiltriert und bei 50°C im Vakuum getrocknet. Auswaage: 395 g S-Phosphoniumsalz S-II; Ausbeute: 70,5 % d.Th. bez. S-VIIb; Fp: 194 bis 195°C; Reinheit nach HPLC: 98,1 %; Drehwert (D, 25°C): +56,18° (C=1 in CH₂Cl₂) Literaturwert für R-II: -57,2° (C=1 in CHCl₃), Helv. Chim. Acta 73, 868 (1990)

Nach Eindampfen der Mutterlauge erhielt man einen Rückstand von 148 g. Durch nochmalige Kristallisation aus Acetonitril/Ethylacetat erhielt man ein Zweitkristallisat von 45,1 g (7,9 % d.Th. bez. S-XII). Fp: 196 bis 197°C; Drehwert (D, 25°C): +52,38° (C=1 in CH₂Cl₂); Reinheit nach HPLC: 93,1 %.

### Beispiel 1n

### Meso-Zeaxanthin

53,2 g (103 mol) Phosphoniumsalz R-II (Drehwert (D) -54,79° (C=1 in CH₂Cl₂) wurden in 250 ml Ethanol gelöst. Man gab 25,0 g (100 mmol) C₁₀-Dialdehyd-mono-neopentylglycolacetal IIIa und 75 ml 1,2-Epoxybutan zu. Anschließend wurde das Gemisch 20h unter Rückfluss nachgerührt. Man ließ auf Raumtemperatur abkühlen und engte am Rotationsverdampfer bei 50°C im Vakuum ein. Der Rückstand (80,3 g) wurde in 300 ml Ethanol gelöst. Man gab eine Lösung von 4,2 g (20 mmol) Citronensäure-monohydrat in 70 ml Wasser zu und rührte bei Raumtemperatur nach. Zur Vervollständigung der Acetalspaltung gab man nach 1 Stunde nochmal 420 mg (2,0 mmol) Citronensäure-monohydrat in 70 ml Wasser zu und rührte bei Raumtemperatur 1 h nach. Dann wurde das Reaktionsgemisch mit 1000 ml Hexan und 500 ml Ethylacetat verdünnt. Man wusch zweimal mit je 100 ml gesättigter Bicarbonatlösung und einmal mit 100 ml gesättigter Kochsalzlösung. Die vereinigten Waschwasserphasen wurden zweimal mit je 200 ml eines 1:1-Gemisches aus Hexan/Ethylacetat reextrahiert.

Die beiden organischen Phasen wurden vereinigt und einmal mit 50 ml gesättigter Kochsalzlösung gewaschen.

Die gesamten vereinigten organischen Phasen wurden über Natriumsulfat getrocknet und am Rotationsverdampfer bei 50°C eingedampft.

Der Eindampfrückstand (70,5 g) wurde in 250 ml Ethanol gelöst. Man gab 75 ml 1,2-Epoxybutan und 53,26 g (103 mmol) Phosphoniumsalz S-II (Drehwert (D, 25°C): +56,18° (C=1 in CH₂Cl₂) zu. Das Gemisch wird 20 h unter Rückfluss nachgerührt. Die entstandene Suspension wurde auf -10°C abgekühlt und 1 h bei -10°C nachgerührt. Das Kristallisat wurde abfiltriert, viermal mit je 100 ml Ethanol gewaschen und im Vakuum bei 50°C getrocknet. Auswaage: 44,6 g meso-Zeaxanthin; Ausbeute: 79 % d.Th. bez. C₁₀-Dial-neopentylglycolacetal; Fp: 207,5 bis 208°C; Reinheit nach UV: 100 %; Reinheit nach HPLC: 98 %; Sterische Einheitlichkeit: > 99 % meso-Zexanthin; Gehalt an R,R-Zeaxanthin und S,S-Zeaxanthin jeweils < 0,3 % [HPLC-Bestimmung nach J. High. Res. Chromatogr. Chrom. Commun. 6, 612 (1989)].

## Patentansprüche

1. Verfahren zur Herstellung von meso-Zeaxanthin, 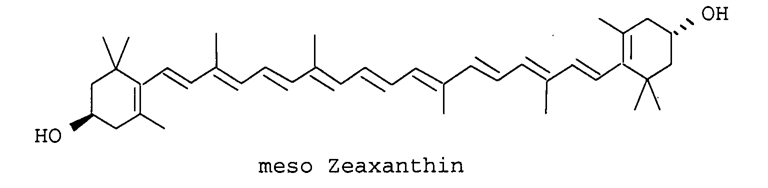 **dadurch gekennzeichnet, dass** man
a) ein racemisches Gemisch der Acetylendiole R-I und S-I 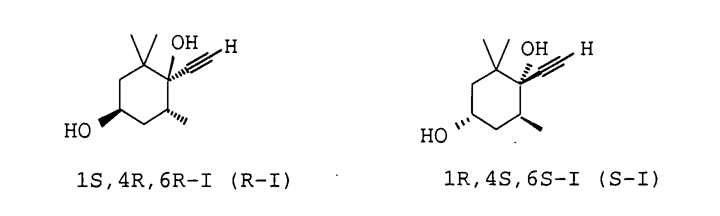 in seine Antipoden auftrennt,
b) die getrennten Antipoden R-I und S-I jeweils in die C₁₅-Phosphoniumsalze R-II bzw. S-II 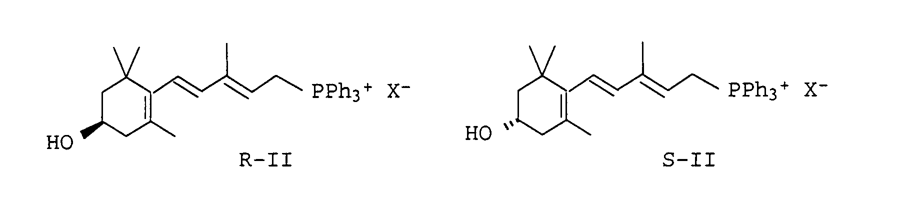 überführt, in denen Ph für Aryl und X für ein Anionenäquivalent einer anorganischen oder organischen Säure stehen,
c) die Phosphoniumsalze R-II oder S-II mit einem C₁₀-Dialmonoacetal der allgemeinen Formel III, 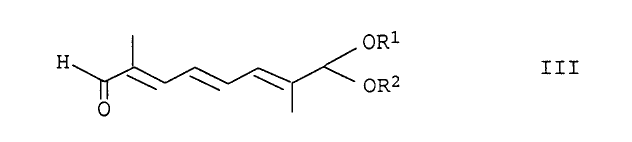 in der die Substituenten R¹ und R² unabhängig voneinander für C₁-C₈-Alkyl stehen oder gemeinsam mit den Sauerstoffatomen und dem Kohlenstoffatom, an denen sie gebunden sind, einen 1,3-Dioxolan- oder 1,3-Dioxan-Ring der folgenden Strukturen bilden können, 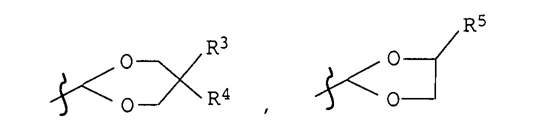 in denen R³ und R⁴ sowie R⁵ jeweils unabhängig voneinander Wasserstoff oder C₁-C₄-Alkyl bedeuten können, in einer Wittig-Reaktion zu den C₂₅-Acetalen R-IV oder S-IV umsetzt, 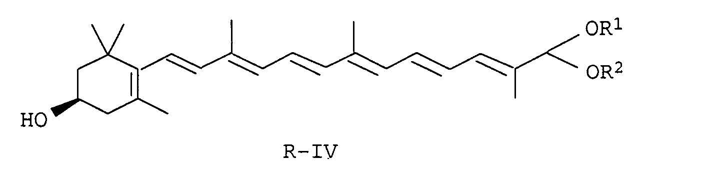 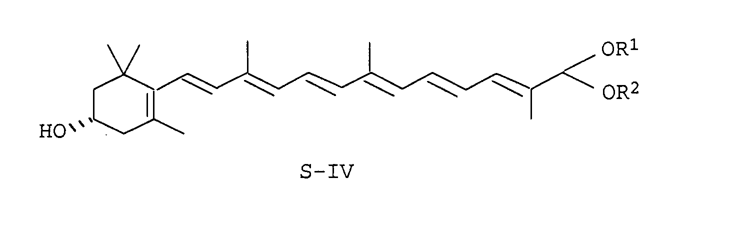
d) die C₂₅-Acetale R-IV oder S-IV in die C₂₅-Aldehyde R-V oder S-V überführt 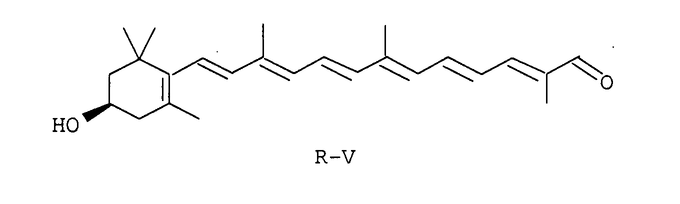 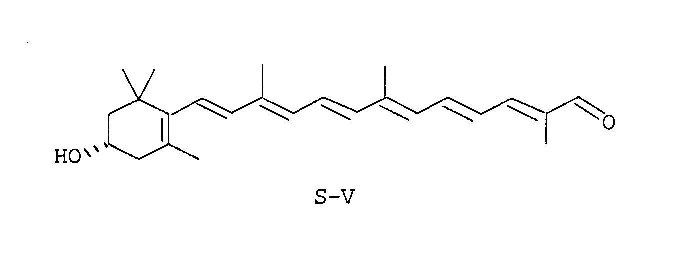
e) und den C₂₅-Aldehyd R-V mit dem C₁₅-Phosphoniumsalz S-II oder den C₂₅-Aldehyd S-V mit dem C₁₅-Phosphoniumalz R-II in einer Wittig-Reaktion zu sterisch einheitlichem meso-Zeaxanthin umsetzt.

2. Verfahren nach Anspruch 1, **dadurch gekennzeichnet, dass** man in der Stufe a) ein racemisches Gemisch der Acetylendiole R-I und S-I unter Verwendung eines optisch aktiven Hilfsreagenzes in ein Diastereomeren-Gemisch überführt, die Diastereomeren trennt und anschließend das Hilfsreagenz wieder abspaltet.

3. Verfahren nach Anspruch 1 oder 2, **dadurch gekennzeichnet, dass** es sich bei dem in der Stufe a) eingesetzten Gemisch um ein diastereomerenreines Racemat der Acetylendiole R-I und S-I handelt.

4. Verfahren nach einem der Ansprüche 1 bis 3, **dadurch gekennzeichnet, dass** man im Verfahrensschritt a) das Racemat selektiv an der sekundären OH-Gruppe mit einem optisch aktiven Hilfsreagenz, ausgewählt aus der Gruppe, bestehend aus Carbonsäuren, Carbonsäurehalogeniden, Chlorcarbonsäureestern, Sulfonsäuren und Isocyanaten zu einem Gemisch diastereomerer Zwischenprodukte der Formeln R-VI und S-VI derivatisiert, 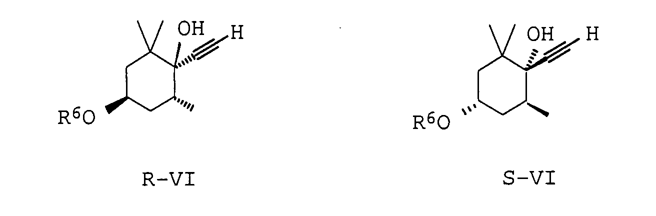 in denen der Substituent R⁶ einen optisch aktiven Urethan-, Carbonat-, Sulfonat- oder einen Acylrest bedeutet.

5. Verfahren nach Anspruch 4, **dadurch gekennzeichnet, dass** man als optisch aktives Hilfsreagenz D- oder L- Milchsäure-Derivate einsetzt.

6. Verfahren nach Anspruch 5, **dadurch gekennzeichnet, dass** man als optisch aktives Hilfsreagenz D-2,4-Dichlorphenoxypropionsäure oder D-2,4-Dichlorphenoxypropionsäurechlorid einsetzt.

7. Verfahren nach einem der Ansprüche 1 bis 6, **dadurch gekennzeichnet, dass** man im Verfahrensschritt a) die diastereomeren Zwischenprodukte durch Kristallisation trennt.

8. Verfahren nach einem der Ansprüche 6 oder 7, **dadurch gekennzeichnet, dass** man aus dem Gemisch der diastereomeren D-2,4-Dichlorphenoxypropionsäureester R-VIa und S-VIa 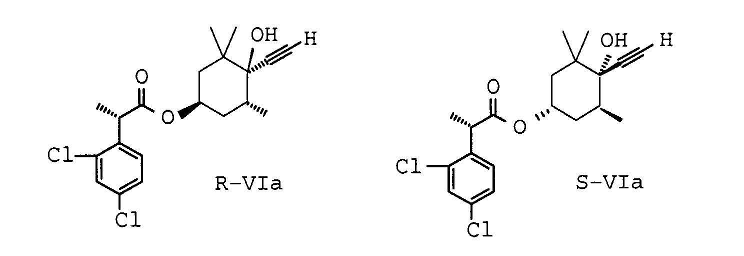 enantiomerenreines R-I durch Kristallisation des Esters und enantiomerenreines S-I durch Kristallisation des hydrolysierten Alkohols erhält.

9. Verfahren nach einem der Ansprüche 1 bis 8, **dadurch gekennzeichnet, dass** man im Verfahrensschritt c) die Phosphoniumsalze R-II oder S-II mit einem C₁₀-Dialdehyd-neopentylglycolmonoacetal der Formel IIIa umsetzt.

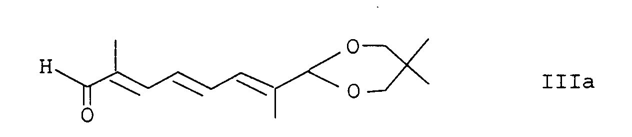

10. Verfahren nach einem der Ansprüche 1 bis 9, **dadurch gekennzeichnet, dass** man die Acetalspaltung im Verfahrensschritt d) in wässrig-ethanolischem Medium unter Zusatz von Zitronensäure als saurem Katalysator durchführt.

11. Verfahren nach einem der Ansprüche 1 bis 10, **dadurch gekennzeichnet, dass** man die Verfahrensschritte c) bis e) ohne Reinigung einer Zwischenstufe durchführt.

12. Verfahren zur Herstellung von optisch reinen Acetylendiolen der Formeln R-I und S-I, 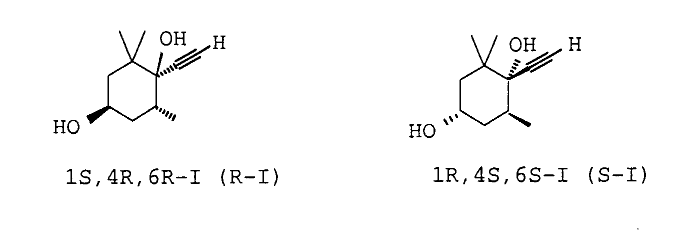 **dadurch gekennzeichnet, daß** man ein racemisches Gemisch der Acetylendiole R-I und S-I unter Verwendung eines optisch aktiven Hilfsreagenzes in ein Diastereomeren-Gemisch überführt, die Diastereomeren trennt und anschließend das Hilfsreagenz wieder abspaltet.

13. Verfahren nach Anspruch 12, **dadurch gekennzeichnet, dass** es sich bei dem Gemisch um ein diastereomerenreines Racemat handelt.

14. Verfahren nach einem der Ansprüche 12 oder 13, **dadurch gekennzeichnet, dass** man das Racemat selektiv an der sekundären OH-Gruppe mit einem optisch aktiven Hilfsreagenz, ausgewählt aus der Gruppe, bestehend aus Carbonsäuren, Carbonsäurehalogeniden, Chlorcarbonsäureestern, Sulfonsäuren und Isocyanaten zu einem Gemisch diastereomerer Zwischenprodukte der Formeln R-VI und S-VI derivatisiert, 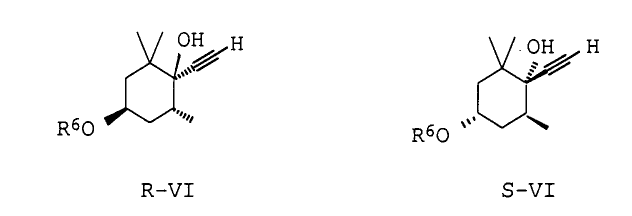 in denen der Substituent R⁶ einen optisch aktiven Urethan-, Carbonat-, Sulfonat- oder einen Acylrest bedeutet.

15. Verfahren nach Anspruch 14, **dadurch gekennzeichnet, dass** man als optisch aktives Hilfsreagenz D- oder L- Milchsäure-Derivate einsetzt.

16. Verfahren nach Anspruch 15, **dadurch gekennzeichnet, dass** man als optisch aktives Hilfsreagenz D-2,4-Dichlorphenoxypropionsäure oder D-2,4-Dichlorphenoxypropionsäurechlorid einsetzt.

17. Verfahren nach einem der Ansprüche 12 bis 16, **dadurch gekennzeichnet, dass** man die diastereomeren Zwischenprodukte durch Kristallisation trennt.

18. Optisch aktive Cyclohexanderivate der allgemeinen Formel R-VI und S-VI, 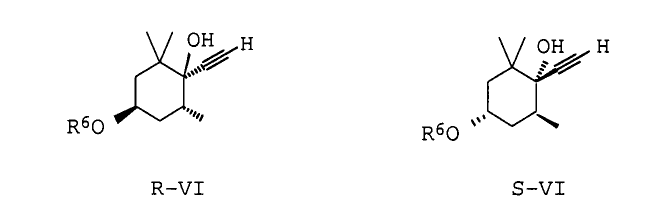 in denen der Substituent R⁶ einen optisch aktiven Urethan-, Carbonat-, Sulfonat- oder einen Acylrest bedeutet.

19. 2,4-Dichlorphenoxypropionsäureester der Formel R-VIa und S-VIa sowie R-VIb und S-VIb

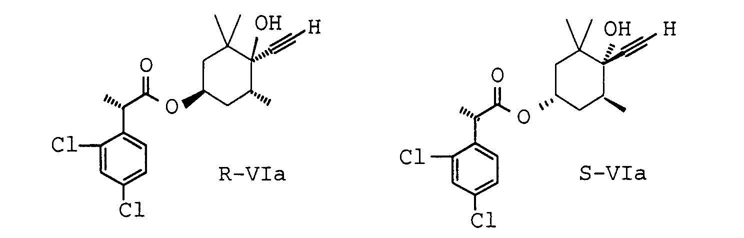

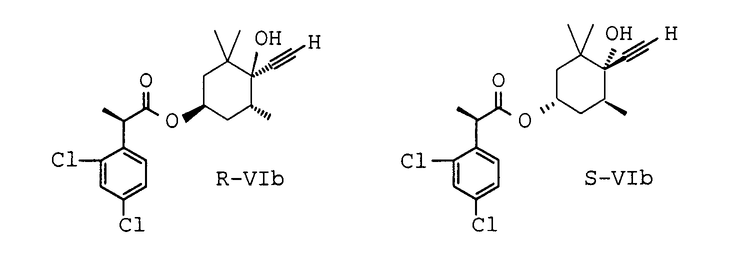

20. Optisch aktives Acetylendiol der Formel S-I

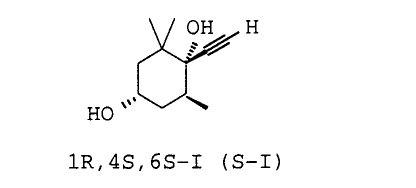

21. Optisch aktive Acetylenverbindungen der allgemeinen Formel S-VII, 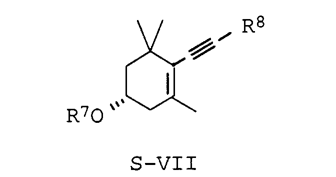 in denen die Substituenten unabhängig voneinander folgende Bedeutung haben:
R⁷ Wasserstoff, C₁-C₁₂-Acyl oder eine hydrolytisch spaltbare Acetal oder Etherschutzgruppe;
R⁸ Wasserstoff oder C(CH₃)OR⁹-CH=CH₂;
R⁹ Lithium oder Wasserstoff.

22. Optisch aktive Cyclohexanderivate der allgemeinen Formel S-XI, 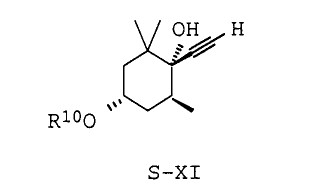 in denen R¹⁰ einen nicht-chiralen C₁-C₁₂-Acylrest oder eine hydrolytisch spaltbare Acetal- oder Etherschutzgruppe bedeutet.
